# EUROPEAN PATENT APPLICATION

(11) **EP 1 859 741 A2**
(43) Date of publication of application: **28.11.2007**
(21) Application number: 07007568.4
(22) Date of filing: 13.04.2007
(51) Int. Cl.: A61B 8/00

(54) **Apparatus and method for displaying an ultrasound image**

(30) Priority: 24.05.2006 KR 20060046603
(71) Applicant: MEDISON CO., LTD., Kangwon-do 250-870 (KR)
(72) Inventor: Kim, Cheol An, Gangnam-gu Seoul 135-280 (KR); Shin, Seong Chul, Gangnam-gu Seoul 135-280 (KR)
(74) Representative: Lorenz, Werner

(57) **Abstract**

Embodiments of the present invention may provide an apparatus and a method of displaying a 3-dimensional ultrasound image formed based on 2-dimensional ultrasound images in an ultrasound diagnostic system. The method of displaying an ultrasound image in an ultrasound diagnostic system, comprises: a) forming a plurality of sequential 2-dimensional ultrasound images based on ultrasound echo signals reflected from a predetermined region of a target object; b) selecting a predetermined number of consecutive 2-dimensional ultrasound images from the 2-dimensional ultrasound images; c) superposing the selected 2-dimensional ultrasound images to form a 3-dimensional ultrasound image; d) setting at least one line on the 3-dimensional ultrasound image; e) cutting the 3-dimensional ultrasound image along the line to obtain a plurality of cutting planes; f) selecting one cutting plane from the cutting planes; and g) rendering the selected cutting plane and displaying the rendered cutting plane.

## Description

The present application claims priority from Korean Patent Application No. 10-2006-0046603 filed on May 24, 2006, the entire subject matter of which is incorporated herein by reference.

### BACKGROUND

### 1. Field

The present invention generally relates to ultrasound image processing, and more particularly to an apparatus and method for displaying a 3-dimensional ultrasound image formed by superposing a plurality of 2-dimensional ultrasound images.

### 2. Background

An ultrasound diagnostic system has become an important and popular diagnostic tool since it has a wide range of applications. Specifically, due to its non-invasive and non-destructive nature, the ultrasound diagnostic system has been extensively used in the medical profession. Modem high-performance ultrasound diagnostic systems and techniques are commonly used to produce two or three-dimensional diagnostic images of internal features of an object (e.g., human organs).

The ultrasound diagnostic system generally uses a wide bandwidth transducer to transmit and receive ultrasound signals. The ultrasound diagnostic system forms images of human internal tissues by electrically exciting an acoustic transducer element or an array of acoustic transducer elements to generate ultrasound signals that travel into the body. The ultrasound signals produce ultrasound echo signals since they are reflected from body tissues, which appear as discontinuities to the propagating ultrasound signals. Various ultrasound echo signals return to the transducer element and are converted into electrical signals, which are amplified and processed to produce ultrasound data for an image of the tissues. The ultrasound diagnostic system is very important in the medical field since it provides physicians with real-time and high-resolution images of human internal features without the need for invasive observation techniques such as surgery.

Generally, the ultrasound diagnostic system obtains raw 3D data (e.g., data on a coordinate system (x, y, z)) through a 3D probe by stacking consecutive frames. It then processes the consecutive frames using a 3D rendering technique, thereby producing 3D static images. By using the static 3D images for ultrasound diagnostic purposes, one may easily and accurately observe, diagnose and treat the internal state of a human body without performing any complicated procedures associated with invasive operations. Thus, the static 3D images are widely used. However, the static 3D images are not useful in observing a moving target object in real time, such as a heart and a fetus in the uterus.

In order to overcome this shortcoming, a live 3D imaging method and apparatus for providing a live 3D moving image (rather than static 3D images) have been developed. However, it is impossible to scan the entire motion of a heart by using a 3-dimensional probe for live 3D imaging due to the limitations in hardware capability. Therefore, there is a problem in that a change of motion at a specific region of the heart cannot be observed in real time. Also, the 3-dimensional probe for forming a live 3D ultrasound image is disadvantageous since it is very complex and expensive.

Accordingly, there is a strong need in the art to form a 3-dimensional ultrasound image in real time without using a 3-dimensional probe.

### BRIEF DESCRIPTION OF THE DRAWINGS

Arrangements and embodiments may be described in detail with reference to the following drawings in which like reference numerals refer to like elements and wherein:

FIG. 1 is a block diagram showing an ultrasound diagnostic system constructed in accordance with the present invention;

FIG. 2 is a flowchart illustrating a method of displaying an ultrasound image in accordance with an embodiment of the present invention;

FIG. 3 is a schematic diagram illustrating a plurality of 2-dimensional ultrasound images formed based on ultrasound echo signals reflected from a predetermined region of a target object;

FIG. 4 is a schematic diagram illustrating a 3-dimensional ultrasound image formed by superposing a plurality of 2-dimensional ultrasound images;

FIG. 5 is a schematic diagram showing a 3-dimensional ultrasound image formed by superposing a plurality of 2-dimensional ultrasound images when movement occurs; and

FIG. 6 is a schematic diagram showing an example of setting an oblique line on the 3-dimensional ultrasound image illustrated in FIG. 5.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

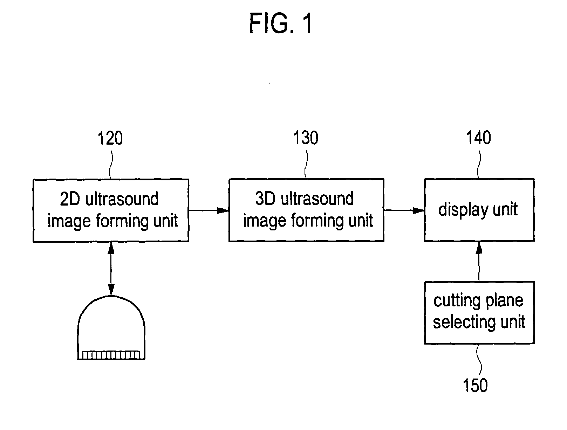

FIG. 1 is a block diagram showing an ultrasound diagnostic system constructed in accordance with the present invention. As shown in FIG. 1, the ultrasound diagnostic system 100 includes a probe 110, a 2-dimensional ultrasound image forming unit 120, a 3-dimensional ultrasound image forming unit 130, a display unit 140 and a cutting plane selecting unit 150. The probe 110 transmits ultrasound signals to a target object and receives ultrasound echo signals. The probe 110 may be any probe capable of acquiring a 2-dimensional ultrasound image.

The 2-dimensional ultrasound image forming unit 120 forms a plurality of 2-dimensional ultrasound images having serial numbers based on the ultrasound echo signals reflected from a predetermined region of a target object. The 2-dimensional ultrasound images may be one of B-mode images, Doppler images and color-mode images. The serial number may be set in an order of acquisition time of the 2-dimensional ultrasound images.

The 3-dimensional ultrasound image forming unit 130 forms a 3-dimensional ultrasound image based on the plurality of 2-dimensional ultrasound images. The 3-dimensional ultrasound image forming unit 130 selects a predetermined number of 2-dimensional ultrasound images in accordance with one embodiment of the present invention. The selected 2-dimensional ultrasound images are sequentially buffered and then superposed, thereby forming the 3-dimensional ultrasound image. Also, the 3-dimensional ultrasound image forming unit 130 may individually render the selected 2-dimensional ultrasound images and can then superpose them in order to form the 3-dimensional ultrasound image.

In accordance with another embodiment of the present invention, the 3-dimensional ultrasound image forming unit 130 may set a region of interest (ROI) in each 2-dimensional ultrasound images and then extracts the images included in the ROIs. The 3-dimensional ultrasound image forming unit 130 can superpose the extracted images, thereby forming the 3-dimensional ultrasound image.

The 3-dimensional ultrasound image forming unit 130 may provide a transparent 3-dimensional ultrasound image obtained by an appropriate transparency treatment for showing a volume of interest (VOI) in the 3-dimensional ultrasound image. The 3-dimensional ultrasound image or the transparent 3-dimensional ultrasound image formed in the 3-dimensional ultrasound image forming unit 130 is transmitted to the display unit and then displayed on a screen of the display unit 140.

The cutting plane selecting unit 150 receives a line setting instruction from a user and sets at least one line on the 3-dimensional ultrasound image displayed on the display unit 140 in response to the line setting instruction. The line setting instruction may be inputted by using a control key (not denoted) provided in the ultrasound diagnostic system to set an arbitrary line on the 3-dimensional ultrasound image. The cutting plane selecting unit 150 cuts the 3-dimensional ultrasound image along the line in a depth direction and then selects one of the cutting planes. The selected plane is rendered, wherein the rendered plane image is then displayed on the display unit 140. A plurality of lines may be set on the 3-dimensional ultrasound image and the rendering of the cutting plane may be performed by using an anti-aliasing technique.

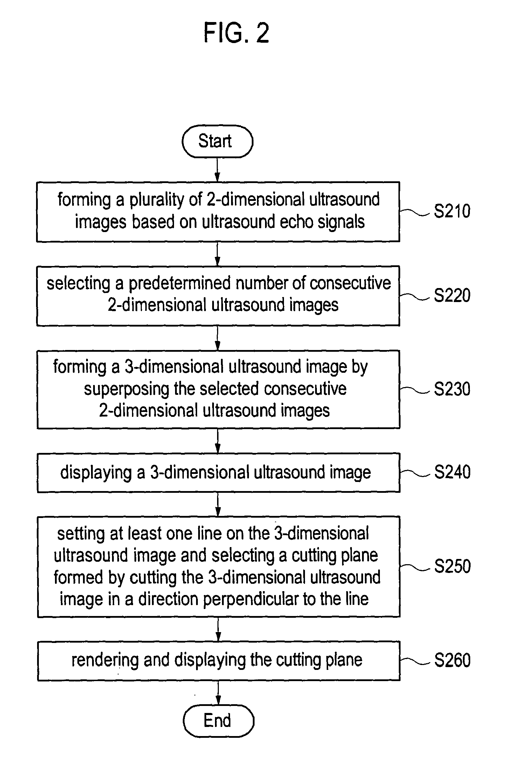

Hereinafter, a method of displaying an ultrasound image will be described in detail with reference to FIGS. 2 to 6. FIG. 2 is a flowchart illustrating a method of displaying the ultrasound image in accordance with one embodiment of the present invention.

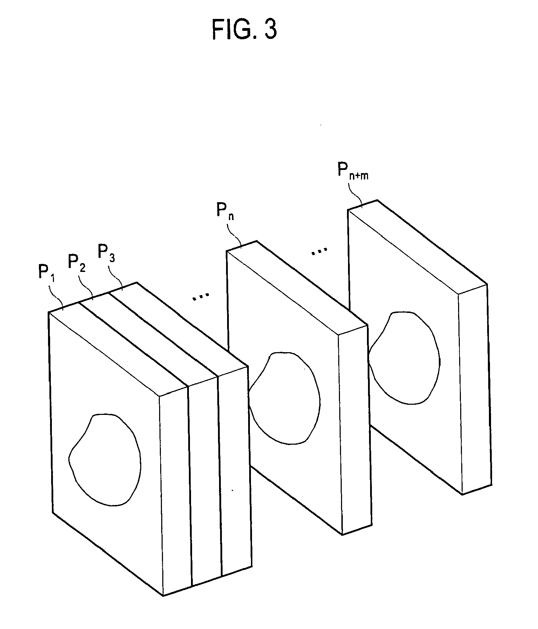

Referring to FIG. 2, the 2-dimensional ultrasound image forming unit 120 forms a plurality of 2-dimensional ultrasound images in real time based on the ultrasound echo signals, which are reflected from a predetermined region of a target object, as shown in FIG. 3 at step S210. Serial numbers P₁-Pₙ₊ₘ may be assigned to each 2-dimensional ultrasound image. The serial numbers assigned to the 2-dimensional ultrasound images may be set in an order of acquisition time of the 2-dimensional ultrasound images. The 3-dimensional ultrasound image forming unit 130 selects a predetermined number of 2-dimensional ultrasound images having consecutive serial numbers among the 2-dimensional ultrasound images at step S220.

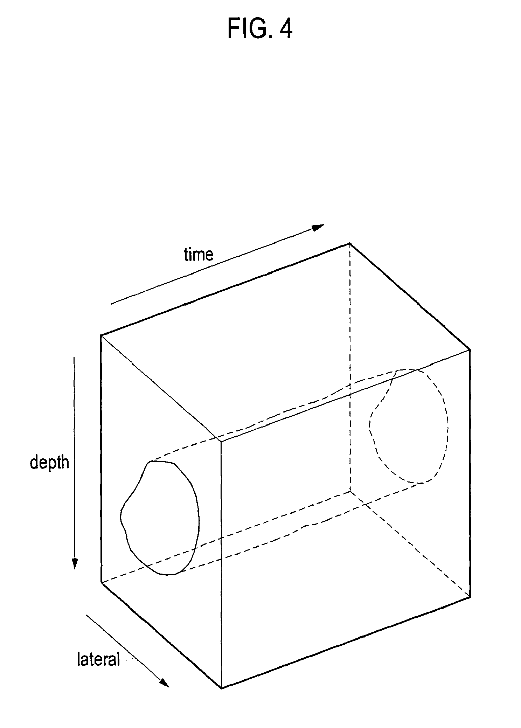

The selected 2-dimensional ultrasound images are superposed to thereby form a 3-dimensional ultrasound image at step S230. The 3-dimensional ultrasound image is displayed on the display unit 140 at step S240, as shown in FIG. 4. Thereafter, at least one line is set on the displayed 3-dimensional ultrasound image and then one cutting plane obtained by cutting the 3-dimensional ultrasound image in a depth direction is selected at step S250. The selected cutting plane is displayed on the display unit 240 at step S260. The cutting plane may be rendered by using the anti-aliasing technique and then displayed on the display unit 140.

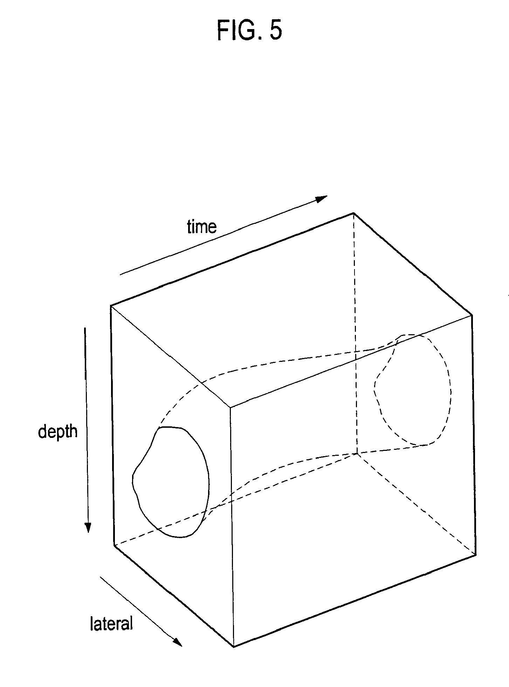

Further, when the 2-dimensional ultrasound images are acquired, the movement of the 2-dimensional ultrasound images may occur due to trembling hands, heart beats or movements of a target object. FIG. 5 is a schematic diagram showing a 3-dimensional ultrasound image formed by superposing 2-dimensional ultrasound images when the movement occurs.

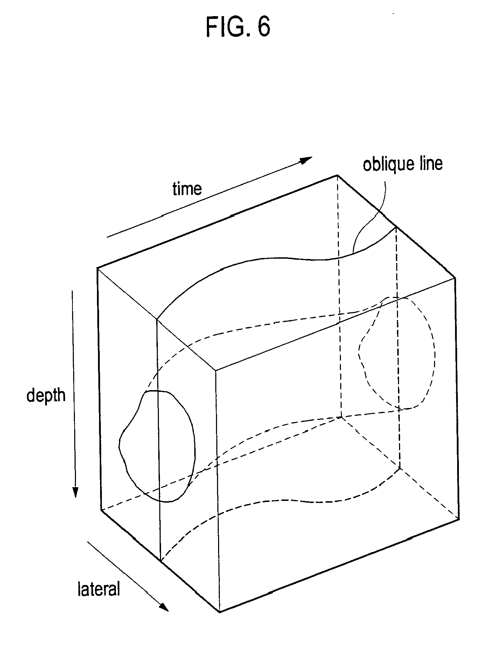

FIG. 6 is a schematic diagram showing an example of setting an oblique line on the 3-dimensional ultrasound image illustrated in FIG. 5. As shown in FIG. 6, an arbitrary oblique line may be set on a desirable portion in the 3-dimensional ultrasound image. The 3-dimensional ultrasound image is then cut along the oblique line in a depth direction. One of the cutting planes may be selected, wherein the selected cutting plane is rendered. The rendered plane is then displayed on the display unit 140.

As mentioned above, the 3-dimensional ultrasound image is formed by superposing the 2-dimensional ultrasound images in accordance with one embodiment of the present invention. As such, the 3-dimensional ultrasound image can be provided without using an expensive 3-dimensional probe.

Also, since the oblique line can be set on the 3-dimensional ultrasound image formed by superposing the 2-dimensional ultrasound images in accordance with the present invention, the user can see the disable plane in the 3-dimensional ultrasound image even if the movement occurs in acquiring the 2-dimensional ultrasound images.

A method of displaying an ultrasound image in an ultrasound diagnostic system, comprises: a) forming a plurality of sequential 2-dimensional ultrasound images based on ultrasound echo signals reflected from a predetermined region of a target object; b) selecting a predetermined number of consecutive 2-dimensional ultrasound images from the plurality of 2-dimensional ultrasound images; c) superposing the selected 2-dimensional ultrasound images to form a 3-dimensional ultrasound image; d) setting at least one line on the 3-dimensional ultrasound image; e) cutting the 3-dimensional ultrasound image along the line to obtain a plurality of cutting planes; f) selecting one cutting plane from the plurality of cutting planes; and g) rendering the selected cutting plane and displaying the rendered cutting plane.

An apparatus of displaying an ultrasound image in an ultrasound diagnostic system, comprises: a probe for transmitting ultrasound signals into a target object and receiving ultrasound echo signals; a 2-dimensional ultrasound image forming unit for forming a plurality of 2-dimensional ultrasound images based on the ultrasound image signals reflected from a predetermined region of a target object; a 3-dimensional ultrasound image forming unit for selecting a predetermined number of consecutive 2-dimensional ultrasound images and superposing the selected 2-dimensional ultrasound images, thereby forming a 3-dimensional ultrasound image; a cutting plane selecting unit for setting at least one line on the 3-dimensional ultrasound image and cutting the 3-dimensional ultrasound image along the line to obtain a plurality of cutting planes, wherein the cutting plane selecting unit is configured to select one cutting plane from the plurality of cutting planes; and a displaying unit for displaying the 3-dimensional ultrasound image and the cutting plane.

Any reference in this specification to "one embodiment," "an embodiment," "example embodiment," etc., means that a particular feature, structure or characteristic described in connection with the embodiment is included in at least one embodiment of the invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. A method of displaying an ultrasound image in an ultrasound diagnostic system, comprising:
a) forming a plurality of sequential 2-dimensional ultrasound images based on ultrasound echo signals reflected from a predetermined region of a target object;
b) selecting a predetermined number of consecutive 2-dimensional ultrasound images from the 2-dimensional ultrasound images;
c) superposing the selected 2-dimensional ultrasound images to form a 3-dimensional ultrasound image;
d) setting at least one line on the 3-dimensional ultrasound image;
e) cutting the 3-dimensional ultrasound image along the line to obtain a plurality of cutting planes;
f) selecting one cutting plane from the cutting planes; and
g) rendering the selected cutting plane and displaying the rendered cutting plane.

2. The method of Claim 1, wherein the step c) includes:
c1) setting a region of interest (ROI) on each selected 2-dimensional ultrasound image;
c2) extracting 2-dimensional ultrasound images included in the ROIs; and
c3) superposing the extracted 2-dimensional images to form the 3-dimesnional ultrasound image.

3. The method of Claim 1, wherein the cutting is carried out along a depth direction.

4. The method of Claim 1, wherein the line set at the step d) includes an oblique line.

5. The method of Claim 1, wherein the rendering is carried out by using an anti-aliasing technique.

6. An apparatus of displaying an ultrasound image in an ultrasound diagnostic system, comprising:
a probe for transmitting ultrasound signals into a target object and receiving ultrasound echo signals;
a 2-dimensional ultrasound image forming unit for forming a plurality of 2-dimensional ultrasound images based on the ultrasound image signals reflected from a predetermined region of a target object;
a 3-dimensional ultrasound image forming unit for selecting a predetermined number of consecutive 2-dimensional ultrasound images and superposing the selected 2-dimensional ultrasound images to thereby form a 3-dimensional ultrasound image;
a cutting plane selecting unit for setting at least one line on the 3-dimensional ultrasound image and cutting the 3-dimensional ultrasound image along the line to obtain a plurality of cutting planes, wherein the cutting plane selecting unit selects one cutting plane from the cutting planes; and
a displaying unit for displaying the 3-dimensional ultrasound image and the cutting plane.

7. The apparatus of Claim 6, wherein the line includes an oblique line.

8. The apparatus of Claim 6, wherein the cutting plane selecting unit cuts the 3-dimensional ultrasound image along a depth direction.

9. The apparatus of Claim 6, wherein the rendering is carried out by using an anti-aliasing method.
